# EUROPEAN PATENT APPLICATION

(11) **EP 3 511 024 A1**
(43) Date of publication of application: **17.07.2019**
(21) Application number: 16883674.0
(22) Date of filing: 24.10.2016
(51) Int. Cl.: A61L 2/10, A01G 31/00, A23L 3/28, B23Q 11/10, C12G 3/02, C12H 1/06

(54) **FLUID STERILIZATION DEVICE AND FLUID STERILIZATION METHOD**

(30) Priority: 06.01.2016 JP 2016001235
(71) Applicant: Nikkiso Co., Ltd., Shibuya-ku, Tokyo 150-6022 (JP)
(72) Inventor: ASANO Hideki, Tokyo 150-6022 (JP); OCHI Tetsumi, Hakusan-shi Ishikawa 924-0004 (JP); KONAGAYOSHI Hidenori, Hakusan-shi Ishikawa 924-0004 (JP); KIUCHI Hiroki, Hakusan-shi Ishikawa 924-0004 (JP)
(74) Representative: Copsey, Timothy Graham
(86) International application number: PCT/JP2016/081422
(87) International publication number: WO 2017/119174

(57) **Abstract**

A fluid sterilization apparatus includes: a flow passage for causing a fluid to flow; and a light source that irradiates the fluid flowing in the flow passage with ultraviolet light of a wavelength of 290∼310 nm. The fluid may contain an aromatic compound. The flow passage may include a straight tube extending in a longitudinal direction, the fluid may flow in the flow passage in a laminal flow state, and the light source may emit ultraviolet light in an intensity distribution in which an ultraviolet light intensity near a center in a cross section of the flow passage perpendicular to a longitudinal direction is higher than an ultraviolet light intensity around.

## Description

### [TECHNICAL FIELD]

The present invention relates to fluid sterilization apparatus and fluid sterilization methods and, more particularly, to a technology of sterilizing a fluid by irradiating the fluid with ultraviolet light.

### [BACKGROUND ART]

It is known that ultraviolet light has sterilization capability. Devices that radiate ultraviolet light are used for sterilization in medical and food processing fronts. Devices that sterilize a fluid such as water continuously by irradiating the fluid with ultraviolet light are also used (see, for example, patent documents 1-3).
[patent document 1] JP4-264199
[patent document 2] JP2012-24880
[patent document 3] JP9-163884

### [PROBLEM TO BE SOLVED BY THE INVENTION]

In related-art fluid sterilization devices, the fluid is irradiated with ultraviolet light of a wavelength of 254 nm emitted from a low-pressure mercury lamp. As pointed out in the patent documents above, the ultraviolet light of this wavelength is absorbed by the fluid itself and only reaches the depth of several millimeters from the surface. Patent document 1 teaches providing a part in the fluid circulation channel where the fluid runs shallow and irradiating the fluid passing through that part with ultraviolet light. This does not, however, provide a radical solution to the issue. A technology for further improving the efficiency of sterilizing a fluid by ultraviolet irradiation is called for.

In this background, one illustrative purpose of the present invention is to provide a technology capable of improving the efficiency of sterilizing a fluid with ultraviolet light.

### [MEANS TO SOLVE THE PROBLEM]

The fluid sterilization apparatus according to an embodiment comprises: a flow passage for causing a fluid to flow; and a light source that irradiates the fluid flowing in the flow passage with ultraviolet light of a wavelength of 290∼310 nm.

According to this embodiment, it is possible, as described below, to inhibit absorption of ultraviolet light by components contained in the fluid and allow the depth of the fluid to be irradiated with ultraviolet light, thereby killing bacteria and viruses contained in the fluid efficiently.

The fluid may contain an aromatic compound. Even in the case of a fluid containing aromatic compounds absorptive of ultraviolet light near 254 nm, bacteria and viruses contained in the fluid are efficiently killed by irradiating the fluid with ultraviolet light of a wavelength of 290∼310 nm.

The flow passage may include a straight tube extending in a longitudinal direction. The fluid may flow in the flow passage in a laminal flow state. The light source may include a light emitting device that emits ultraviolet light of a wavelength of 290∼310 nm and radiate ultraviolet light in an intensity distribution in which an ultraviolet light intensity near a center in a cross section of the flow passage perpendicular to the longitudinal direction is higher than an ultraviolet light intensity around.

According to this embodiment, the fluid flowing in a laminal flow state is irradiated with ultraviolet light so that the sterilization efficiency is improved as compared to the case of irradiating the fluid in a turbulent state with ultraviolet light. Our knowledge shows that the sterilization efficiency in a laminal flow state is about seven times as great as that of a turbulent flow state in the case of irradiating the interior of a flow passage of a straight tube shape with ultraviolet light for sterilization. Since a fluid in a laminal flow state has a flow rate distribution in which the flow rate near the center is higher and the flow rate near the tube wall is lower, the fluid flowing in the flow passage is irradiated effectively with ultraviolet light and the sterilization efficiency is improved, by increasing the ultraviolet light intensity near the center in association with the flow rate distribution.

The fluid may contain a cutting fluid for a cutting work. According to this embodiment, bacteria floating in the air are prevented from proliferating, when dissolved in the cutting fluid during a cutting work, on culture media of organic components contained in the cutting fluid.

The fluid may contain a culture fluid for hydroponic culture. According to this embodiment, pathogenic bacteria mixed in a culture fluid for hydroponic culture are prevented from proliferating and causing the plant to perish.

The fluid may contain Japanese sake in the process of manufacturing. According to this embodiment, lactic acid bacteria that survive the manufacturing step of Japanese sake are prevented from fermenting the sugar contained in Japanese sake and impairing the original taste of Japanese sake.

Another embodiment of the present invention relates to a fluid sterilization method. The method comprises: irradiating a fluid flowing in a flow passage with ultraviolet light of a wavelength of 290∼310 nm.

According to this embodiment, it is equally possible to inhibit absorption of ultraviolet light by components contained in the fluid and allow the depth of the fluid to be irradiated with ultraviolet light, thereby killing bacteria and viruses contained in the fluid efficiently.

### [ADVANTAGE OF THE INVENTION]

According to the present invention, the efficiency of sterilizing a fluid with ultraviolet light is improved.

### [BRIEF DESCRIPTION OF THE DRAWINGS]

Fig. 1 shows an example of transmission spectrum of a cutting fluid exemplifying a fluid;
Fig. 2 shows absorption spectra of benzene, naphthalene, and anthracene by way of example of organic compounds having a conjugate double bond;
Fig. 3 shows absorption spectra of phenol and p-nitrophenol exemplifying aromatic compounds having a functional group, along with an absorption spectrum of benzene;
Fig. 4 shows an example of transmission spectrum of Japanese sake exemplifying a fluid;
Fig. 5 shows an example of wavelength dependence of sterilization effect by ultraviolet light;
Fig. 6 is a cross-sectional view schematically showing a configuration of a fluid sterilization apparatus according to the first embodiment;
Fig. 7 is a contour figure showing an ultraviolet light intensity distribution in the flow passage;
Fig. 8 is a contour figure showing a flow rate distribution of a fluid in a turbulent state;
Fig. 9 is a contour figure showing a flow rate distribution of a fluid in a laminal flow state;
Fig. 10 is a front view schematically showing a configuration of a light source according to a variation;
Fig. 11 is a cross-sectional view schematically showing a configuration of a fluid sterilization apparatus according to the second embodiment; and
Fig. 12 is a cross-sectional view schematically showing a configuration of the fluid sterilization apparatus of Fig. 11.

### [MODE FOR CARRYING OUT THE INVENTION]

The fluid sterilization apparatus according to an embodiment irradiates a fluid with ultraviolet light of a relatively long wavelength (e.g., wavelength in a range of 290∼310 nm) instead of ultraviolet light of a wavelength near 254 nm, which is used in related-art fluid sterilization devices. This inhibits absorption of ultraviolet light by components contained in the fluid and allows the depth of the fluid to be irradiated with ultraviolet light, thereby killing bacteria and viruses contained in the fluid efficiently. A description will be given of the reason why ultraviolet of a wavelength in the range of 290∼310 nm should be radiated.

### [Fluid subject to sterilization]

In machine tools such as a turning machine, a coolant containing ethylene glycol as a main component is widely used as a cutting fluid. The coolant has a function of a lubricant and a cooling agent at a working point. The inevitable rise in the temperature of the coolant itself with use creates an environment in which bacteria floating in the air are prone, when dissolved in the coolant, to proliferate on culture media of organic components contained in the coolant. As a result, a terrible smell is given off during work especially in summer, resulting in a poor working environment. Components produced by bacteria lower the pH of the fluid and cause rust or corrosion of the worked product and working machine, causing problems like quality loss and reduced life of the working machine. Growth of mold in the cutting fluid is also a serious problem. Growth of mold results in adhesion of deposited material on the wall of the liquid tank. The deposited material inhibits the performance of the coolant and, particularly, lowers the performance of the filtering system. Use of a disinfectant for fungus to control the growth of mold may lead to a change in the cutting performance and so is not preferable especially in a working machine in which high accuracy is required. For this reason, sterilization of the cutting fluid with ultraviolet light is called for.

In hydroponic culture and plant factories, a culture fluid necessary for growth is circulated. Often, the culture fluid is infiltrated by pathogenic bacteria. Once pathogenic bacteria are mixed in the culture fluid, the plant may perish totally because the culture fluid is circulated. In vegetable cultivation, pesticides are often used to combat diseases. In the case of hydroponic culture, however, pesticides, if used, are dissolved in the culture fluid and contaminate the inner part of the plant. Therefore, most pesticides are unsuitable for use in hydroponic culture. For this reason, sterilization of the culture fluid with ultraviolet light is called for.

Japanese sake is an alcoholic beverage made by fermenting rice with koji. Lactic acid bacteria that survive the manufacturing step ferment the sugar contained in Japanese sake rapidly, with the result that the original taste of Japanese sake is significantly impaired. For this reason, a step called "hi-i-ire", whereby koji contained in the fermented, unprocessed sake is removed by a filter, and the filtered sake is then processed at a temperature of about 60°, is performed in the production of Japanese sake. Lactic acid bacteria are killed in this step. However, "hi-i-re" is known to impair the rich flavor and taste that Japanese sake has. Attempts are therefore made to prevent the flavor from being deteriorated by using a low temperature, shorten the time or the like. Therefore, lactic acid bacteria may remain in very rare circumstances. In that case, lactic acid bacteria proliferate before the product reaches the consumer via a distribution channel. By the time a consumer opens the bottle, the sake will contain a carbon dioxide gas and turn into an acetic taste. The phenomenon is called "hi-ochi". Killing of lactic acid bacteria with ultraviolet light makes "hi-i-re" unnecessary and is believed to make it possible to manufacture Japanese sake of mallow taste in which the flavor and taste are not impaired.

### [Ultraviolet light absorption property of fluid]

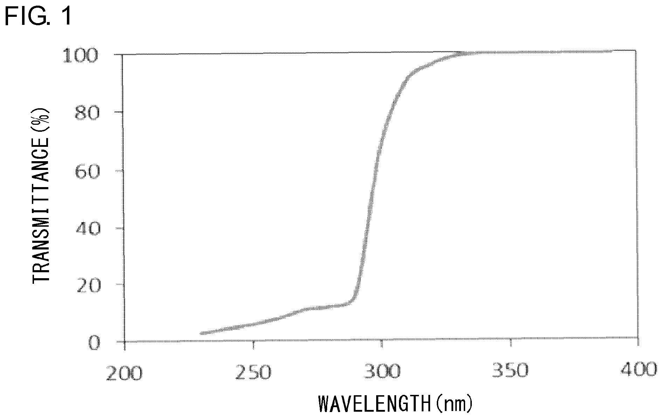

Fig. 1 shows an example of transmission spectrum of a cutting fluid exemplifying a fluid. The transmission spectrum is measured by using a commercially available cutting fluid. The spectrum shows that transmittance of ultraviolet light of a wavelength less than about 290 nm is as low as 15% or below but ultraviolet light of a wavelength of about 290 nm or more is transmitted without being absorbed as much. Such transmission property is considered to derive from absorption of ultraviolet light below about 290 nm by the following organic compounds added to the cutting fluid.

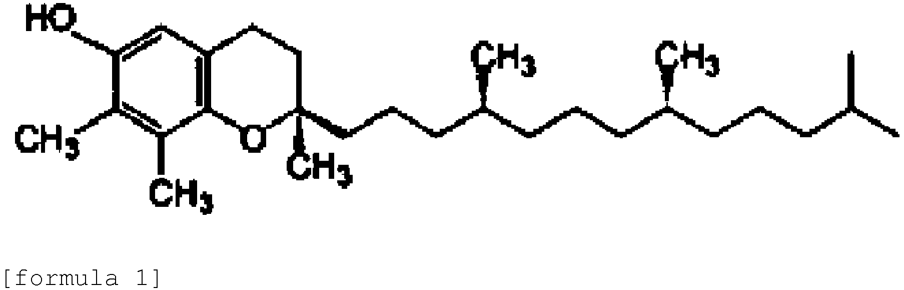

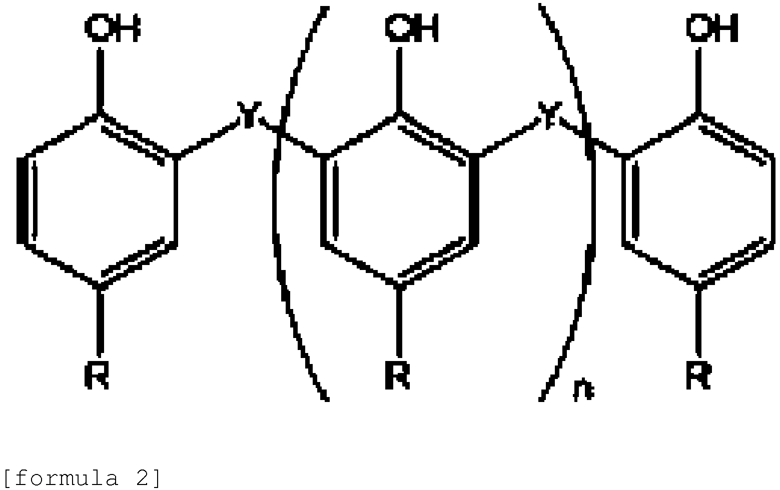

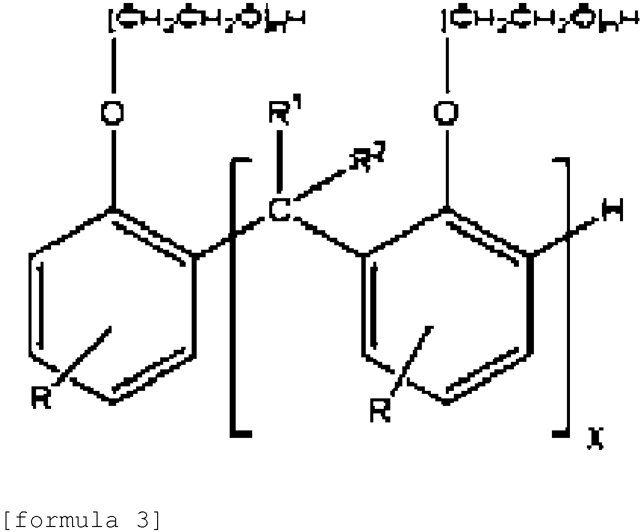

All these organic compounds contain a benzene ring, and it is considered that the presence of conjugate double bonds shifts the wavelength of absorbed ultraviolet light toward the longer wavelength side with the result that ultraviolet light of a wavelength up to about 290 nm is absorbed.

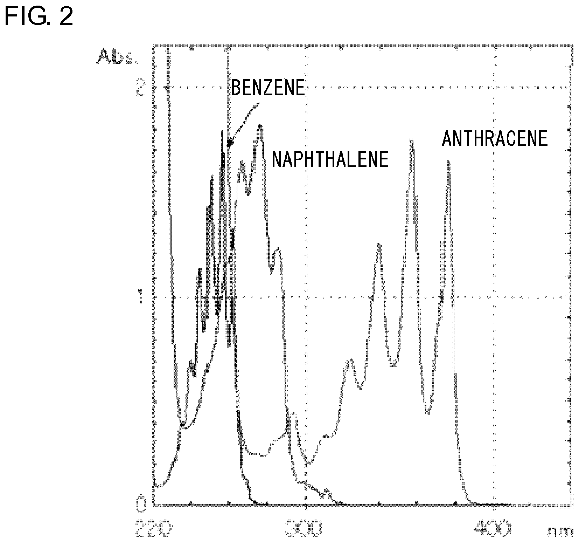

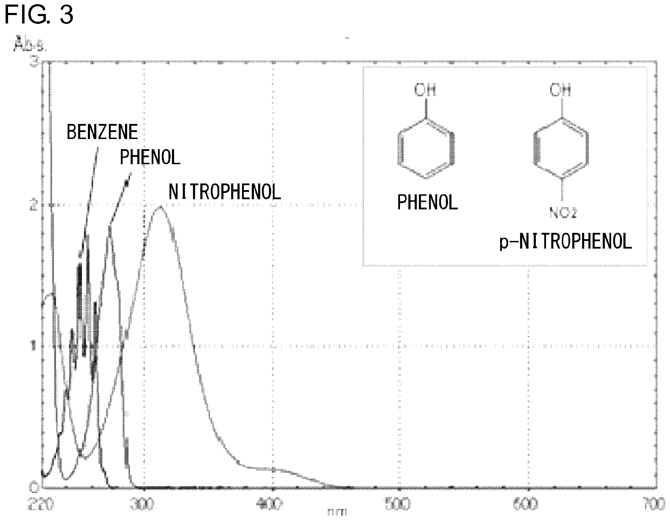

It is well known that with the increase in conjugate double bonds, the wavelength of absorbed ultraviolet light is shifted toward the longer wavelength side. Fig. 2 shows absorption spectra of benzene, naphthalene, and anthracene by way of example of organic compounds having a conjugate double bond. The rising edge of absorption is shifted to near 270 nm in the case of benzene, about 310 nm in the case of naphthalene, and 390 nm in the case of anthracene. Further, the wavelength of ultraviolet light absorbed is also affected by the functional group bonded to the benzene ring. Fig. 3 shows absorption spectra of phenol and p-nitrophenol exemplifying aromatic compounds having a functional group, along with an absorption spectrum of benzene. The hydroxyl group and nitro group shift absorption in phenol and p-nitrophenol more toward the longer wavelength side than in the case of benzene.

The fluid such as a cutting fluid contains aromatic compounds as additives or impurities. Aromatic compounds are generally highly absorptive of ultraviolet light of a wavelength near 254 nm. Therefore, most of ultraviolet light of a wavelength near 254 nm irradiating the fluid will be absorbed by aromatic compounds near the surface of the fluid.

By way of contrast, ultraviolet light of a wavelength longer than 254 nm, and, for example, 290 nm or longer, is hardly absorbed by benzene, naphthalene, or phenol.

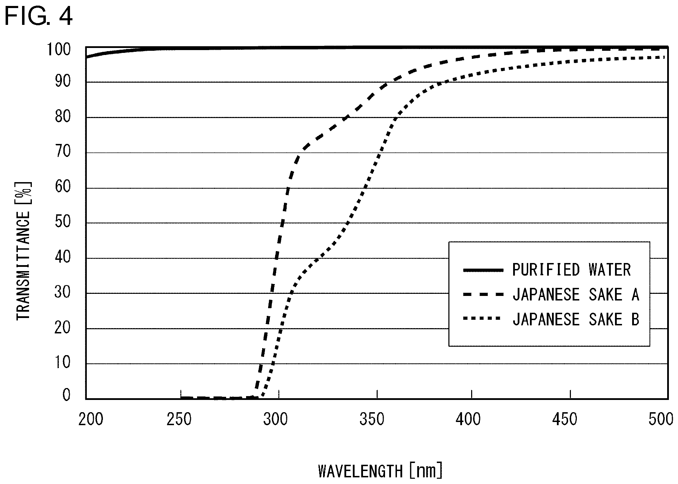

Fig. 4 shows an example of transmission spectrum of Japanese sake. The graph shows transmission spectra of two kinds of commercially available Japanese sake and purified water. In each Japanese sake, hardly any light is transmitted in a wavelength region shorter than 285 nm. This is considered to be because Japanese sake contains phenol in an amount of about 10 ppm∼300 ppm and a large amount of aromatic amino acids so that these aromatic compounds absorb most of ultraviolet light of a wavelength less than 285 nm.

### [Wavelength dependence of sterilization effect by ultraviolet light]

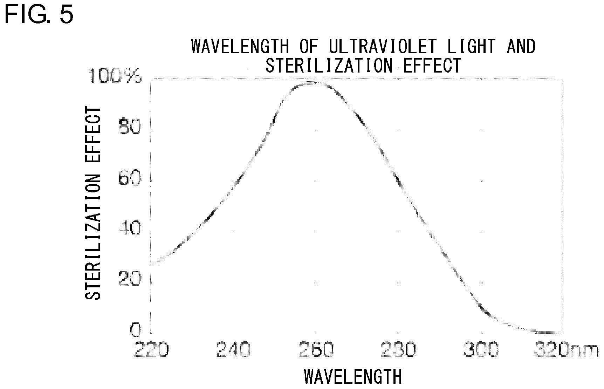

Fig. 5 shows an example of wavelength dependence of sterilization effect by ultraviolet light. Nucleic acids in bacteria and viruses are said to absorb ultraviolet light of a wavelength near 260 nm efficiently as shown in the figure. Therefore, as described above, related-art fluid sterilization devices are configured to irradiate the fluid with ultraviolet light of a wavelength near 254 nm. As shown in the graph, however, ultraviolet light of longer wavelengths is expected to provide sterilization effect given that the wavelength is up to about 310 nm. Ultraviolet light in this range is less effective than ultraviolet light of a wavelength near 254 nm. However, the deficiency can be cured by increasing the light amount or time of irradiation. For example, a fluid can be sterilized properly by irradiating it with ultraviolet light of a wavelength of, for example, 290∼310 nm. The sterilization effect by irradiation with ultraviolet light of a wavelength of near 290 nm is about 30%, meaning a drop of about 1/3 from the case of irradiation with ultraviolet light of a wavelength near 254 nm characterized by the greatest sterilization effect. However, the equivalent sterilization effect can be obtained by increasing the ultraviolet irradiation energy about three times. This is a sufficiently practical value in terms of application to the fluid sterilization apparatus.

### [Wavelength of ultraviolet light that should be radiated]

As described above, it is preferable to radiate ultraviolet light of a wavelength of 290 nm or longer in order to reduce absorption of ultraviolet light by aromatic compounds contained in the fluid subject to sterilization, and it is preferable to radiate ultraviolet light of a wavelength of 310 nm or shorter in order to kill bacteria or viruses contained in the fluid effectively.

The upper limit value may be selected in accordance with the types of organic compounds contained in the fluid. For example, ultraviolet light of a wavelength of 270 nm or longer may be used if the main component is benzene and the other aromatic compounds are contained in very little amount. Ultraviolet light of a wavelength of 270 nm or longer may also be used in the case where the main component is anthracene. If the main component is naphthalene or phenol, it is preferable to use ultraviolet light of a wavelength of 290 nm or longer. If the main component is p-nitrophenol, on the other hand, it is preferable to use ultraviolet light of a wavelength of 300 nm or shorter, and, more preferably, 280 nm or shorter. The absorption spectrum of the fluid subject to sterilization may be measured beforehand to determine the wavelength of ultraviolet light that should be radiated. In this case, the permitted absorption level of the fluid may be determined in consideration of the amount of ultraviolet light radiated, duration of irradiation, irradiation energy, and depth of the channel at the position of ultraviolet irradiation, and the wavelength of ultraviolet light radiated may be determined based on the absorption level thus determined.

The upper limit value may be selected in accordance with the types of bacteria and viruses contained in the fluid. For example, if bacteria and viruses for which ultraviolet light of a longer wavelength than the example shown in Fig. 5 is effective are contained in the fluid, ultraviolet light of a wavelength longer than 310 nm may be used. For example, the upper limit value may be 320 nm, 330 nm, 340 nm, or 350 nm. In this case, too, the types of bacteria and viruses contained in the fluid subject to sterilization may be examined beforehand and the wavelength of ultraviolet light may be determined based on the types of bacteria and viruses contained.

### [Configuration of the fluid sterilization apparatus]

The light source for irradiating the fluid with ultraviolet light of the wavelength discussed above is exemplified by a xenon lamp, deuterium lamp, and light emitting diode. A xenon lamp and a deuterium lamp emit light of wavelengths other than the wavelength(s) discussed above and make it necessary to use a filter to prevent the temperature of the fluid from increasing due to the heat ray. Accordingly, it is preferable to use a light emitting diode capable of emitting ultraviolet light of the wavelength discussed above selectively. A description will now be given of a configuration of a fluid sterilization apparatus in which a light emitting diode is used as a light source. Like numerals are used in the description to denote like elements and the description is omitted as appropriate.

### (First embodiment)

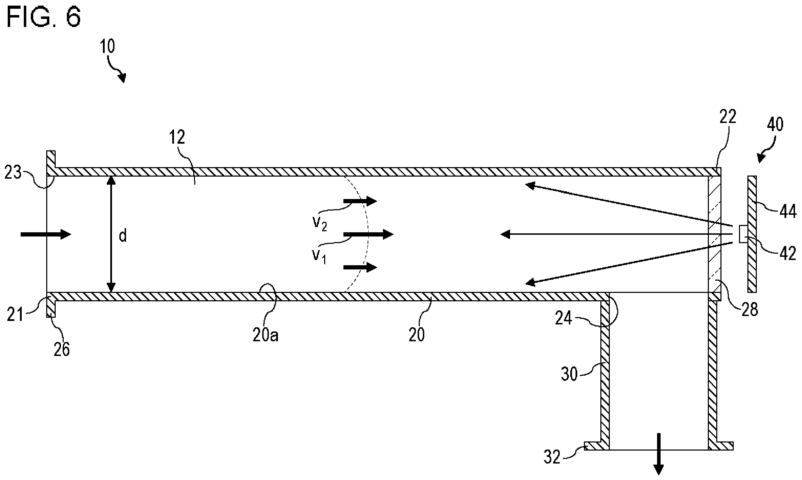

Fig. 6 schematically shows a configuration of a fluid sterilization apparatus 10 according to the first embodiment. The fluid sterilization apparatus 10 includes a straight tube 20, an outflow pipe 30, and a light source 40. The light source 40 is positioned at an end (second end 22) of the straight tube 20 and radiates ultraviolet light toward the interior of the straight tube 20. The fluid sterilization apparatus 10 is used to irradiate a fluid (water etc.) flowing in the straight tube 20 with ultraviolet light so as to sterilize the fluid.

The straight tube 20 includes a first end 21, a second end 22, a first flange 36, and a window 28. The straight tube 20 extends from the first end 21 to the second end 22 in the longitudinal direction. The first end 21 is provided with an inflow port 23 for causing the fluid to flow in in the longitudinal direction of the straight tube 20 and a first flange 26 for connecting the inflow port 23 to another pipe, etc. A window 28 for transmitting the ultraviolet light from the light source 40 is provided on the second end 22. The window 28 is made of a material having a high ultraviolet transmittance such as quartz (SiO2), sapphire (Al2O3), and amorphous fluororesin.

The second end 22 is provided with an outflow port 24 for causing the fluid to flow out in a direction intersecting or perpendicular to the longitudinal direction of the straight tube 20. The outflow port 24 is provided on the side wall of the straight tube 20, and an outflow pipe 30 is fitted to the outflow pipe 30. One end of the outflow pipe 30 is fitted to the outflow port 24, and a second flange 32 is provided at the other end. Therefore, the straight tube 20 and the outflow pipe 30 form an L-shaped flow passage 12. The fluid flowing in via the first flange 26 flows through the inflow port 23, the straight tube 20, the outflow port 24, and the outflow pipe 30 before flowing out via the second flange 32.

The straight tube 20 and the outflow pipe 30 are made of a metal material or a resin material. An inner wall surface 20a of the straight tube 20 is desirably made of a material having a high ultraviolet reflectivity. For example, the inner wall surface 20a is made of mirror-polished aluminum (Al) or polytetrafluoroethylene (PTFE), which is a fully fluorinated resin. By forming the inner wall surface 20a of the straight tube 20 using a material like the above, the ultraviolet light emitted by the light source 40 can be reflected by the inner wall surface 20a to propagate in the longitudinal direction of the straight tube 20. PTFE is a chemically stable material and has a high ultraviolet reflectivity and so is suitable as the material of the straight tube 20 forming the fluid sterilization apparatus.

The inner diameter d of the straight tube 20 and the average flow rate v of the fluid flowing in the flow passage 12 are adjusted so that the fluid flowing in the flow passage 12 is in a laminal flow state. More specifically, the inner diameter d and the average flow rate v are adjusted such that the Reynolds number Re of the flow passage 12 is equal to or less than the critical Reynolds number of the laminal flow, using an expression Re=v^{∗}d/v(v: dynamic coefficient of viscosity). The value equal to or less than the critical Reynolds number means that the Reynolds number Re is 3000 or less, and, preferably, 2500 or less, and more preferably, 2320 or less. Further, by causing the fluid to flow from the inflow port 23 into the flow passage 12 in the longitudinal direction, the fluid is caused to flow toward the second end 22 in a laminal flow state. When the fluid flows in a laminal flow state, the flow rate distribution is such that the flow rate v1 of the fluid flowing near the central axis of the straight tube 20 is relatively high and the flow rate v2 of the fluid flowing near the inner wall surface 20a of the straight tube 20 is relatively low. In an ideal laminal flow state, the fluid flowing in the flow passage 12 presents a flow rate distribution given by an expression of a paraboloid of revolution.

The light source 40 includes a light emitting device 42 and a substrate 44. The light emitting device 42 is a light emitting diode (LEDs) configured to emit ultraviolet light, and the central wavelength or peak wavelength thereof is included in a range of about 200 nm∼350 nm. It is preferable that the light emitting device 42 emit ultraviolet light in the aforementioned wavelength range (e.g., near 290 nm∼310 nm). Such an ultraviolet LED is exemplified by an aluminum gallium nitride (AlGaN) based LED.

The light emitting device 42 is an LED having a predetermined directivity angle or light distribution angle. For example, the light emitting device 42 is a wide light-distribution LED characterized by a light distribution angle (full-angle value) of 120° or more. The light emitting device 42 with such a specification is exemplified by an LED of a surface mount device (SMD) type characterized by a high output intensity. The light emitting device 42 is arranged on the central axis of the straight tube 20 and is fitted to the substrate 44 so as to face the window 28. The substrate 44 is made by using a highly exoergic member. For example, copper (Cu), aluminum (Al), or the like is used as a base material. The heat generated by the light emitting device 42 is dissipated via the substrate 44.

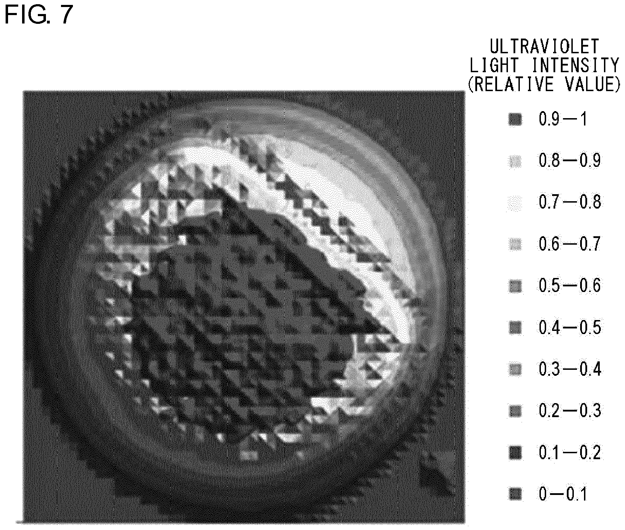

Fig. 7 is a contour figure showing an ultraviolet light intensity distribution in the flow passage 12. Since the light emitting device 42 emits ultraviolet light having a predetermined light distribution angle, the intensity distribution is such that the ultraviolet light intensity near the center is higher than the ultraviolet light intensity around. As a result, the ultraviolet light intensity distribution in the straight tube 20 is such that the ultraviolet light intensity near the central axis is higher and the ultraviolet light intensity near the inner wall surface 20a is lower in a cross-sectional view of the flow passage 12 perpendicular to the longitudinal direction.

With the above features, the fluid sterilization apparatus 10 irradiates the fluid flowing in the straight tube 20 with ultraviolet light to sterilize the fluid. The ultraviolet light from the light source 40 is radiated such that the intensity is higher near the center of the straight tube 20 and lower near the inner wall surface 20a of the straight tube 20. The fluid is caused to flow in the flow passage 12 such that the flow rate v1 near the center is higher and the flow rate v2 near the inner wall surface 20a is lower. As a result, a laminal flow state is produced so that the amount of energy of ultraviolet light affecting the fluid passing through the straight tube 20 is uniformized regardless of the radial position where the fluid passes. This allows the entire fluid flowing in the straight tube 20 to be irradiated with ultraviolet of a predetermined amount of energy or higher and enhances the sterilization effect on the entire fluid.

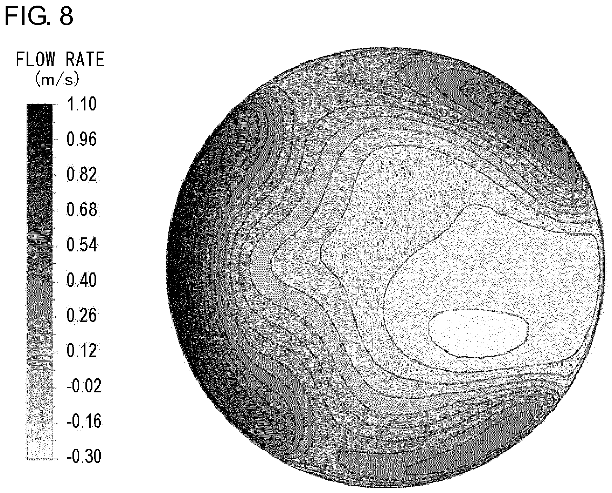

A description will now be given of the advantage of the fluid sterilization apparatus 10 with reference to a comparative example. Fig. 8 is a contour figure showing a flow rate distribution of a fluid in a turbulent state and shows a flow rate distribution occurring when the fluid is caused to flow in the straight tube 20 in a condition in which the Reynolds number Re=4961. In the illustrated example, the flow rate in some portions near the inner wall surface 20a is the highest, and the flow rate near the center is of a negative value. The flow rate distribution of the fluid is not constant and changes with time. A fungus liquid containing Escherichia coli is caused to flow in a condition in which a turbulent flow state like this is produced. The survival rate of Escherichia coli contained in the processed fluid was found to be 0.53%.

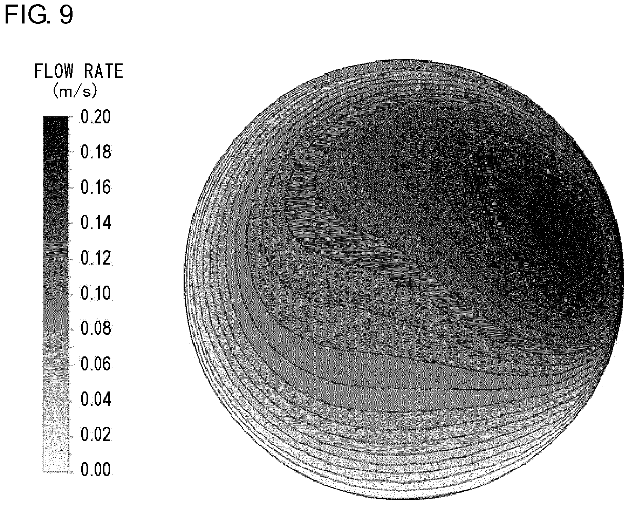

Fig. 9 is a contour figure showing a flow rate distribution of a fluid in a laminal flow state and shows a flow rate distribution occurring when the fluid is caused to flow in the straight tube 20 in a condition in which the Reynolds number Re=2279. In the illustrated example, the portion with the highest flow rate is shifted toward top right, but the flow rate distribution is generally such that the flow rate near the center is higher and the flow rate near the inner wall surface 20a is lower. A fungus liquid containing Escherichia coli is caused to flow in a condition in which a lamina flow state like this is produced. The survival rate of Escherichia coli contained in the processed fluid was found to be 0.07%. These results reveal that the sterilization effect in a laminal flow state is about seven times as great as that of a turbulent flow state. Thus, according to this embodiment, the fluid in a laminal flow state is irradiated with ultraviolet light of an intensity distribution corresponding to the flow rate distribution of the laminal flow state. Therefore, the sterilization efficiency for the fluid is improved.

In accordance with this embodiment, the inflow port 23 and the light source 40 are positioned on the central axis of the straight tube 20 so that a smooth flow of the fluid is created in the direction of irradiation with the ultraviolet light from the light source 40. By providing the inflow port 23 at a position opposite to the light source 40, the fluid that is made less turbulent and turned into a laminal flow state by traveling in the straight tube 20 is irradiated with ultraviolet light with a great intensity. This can inhibit unevenness in the amount of ultraviolet energy radiated, created as a portion of the fluid passes through a location where the ultraviolet light intensity is low at a high speed or a portion of the fluid is turned into a stagnant eddy at a location where the ultraviolet light intensity is high, and inhibit the associated impact of lower sterilization effect.

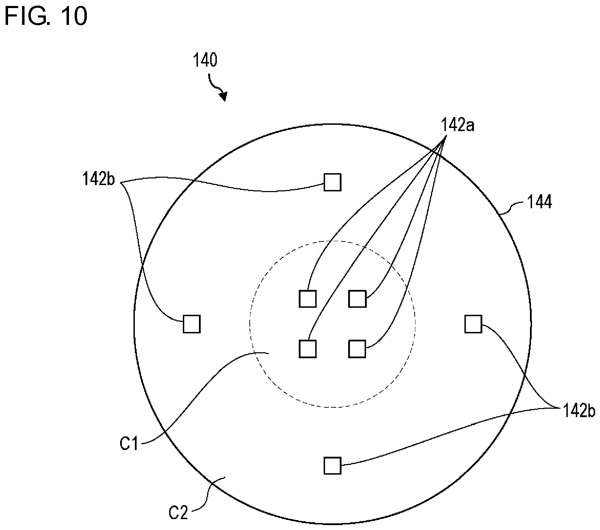

Fig. 10 is a front view schematically showing a configuration of a light source 140 according to a variation. The light source 140 includes a plurality of light emitting devices 142a, 142b, and a substrate 144. The light source 140 includes a plurality of first light emitting devices 142a positioned close to each other in a central region C1 of the substrate 144 and a plurality of second light emitting devices 142b scattered in a peripheral region C2 of the substrate 144. The first light emitting devices 142a and the second light emitting devices 142b are configured similarly to the light emitting device 42 described above.

Since the first light emitting devices 142a are positioned close to each other in the central region C1, the light source 140 outputs ultraviolet light of a relatively high intensity in the central region C1. Meanwhile, the second light emitting devices 142b are positioned sparsely in the peripheral region C2 so that the light source 140 outputs ultraviolet light of a relatively low intensity in the peripheral region C2. Therefore, by applying the light source 140 according to this variation to the fluid sterilization apparatus 10 described above, the fluid can be irradiated with ultraviolet light having an ultraviolet light intensity in which the ultraviolet light intensity is higher near the center and the ultraviolet light intensity is lower near the inner wall surface 20a even when the diameter d of the straight tube 20 is increased to increase the processing volume.

### (Second embodiment)

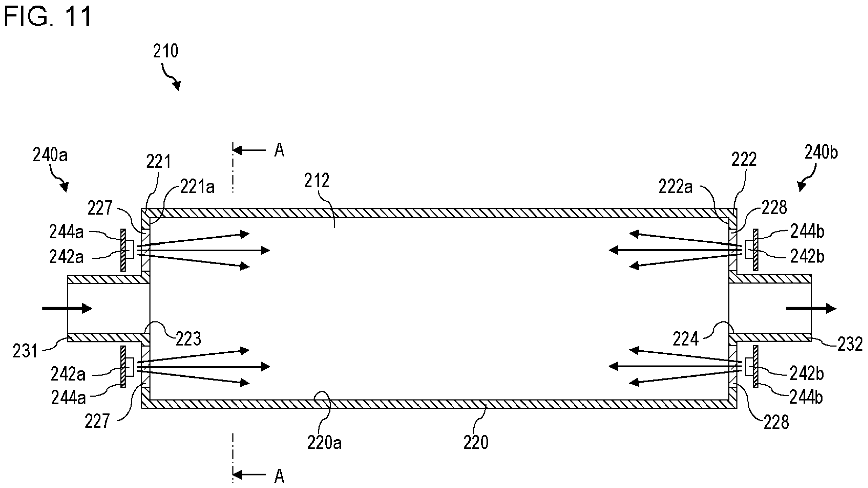

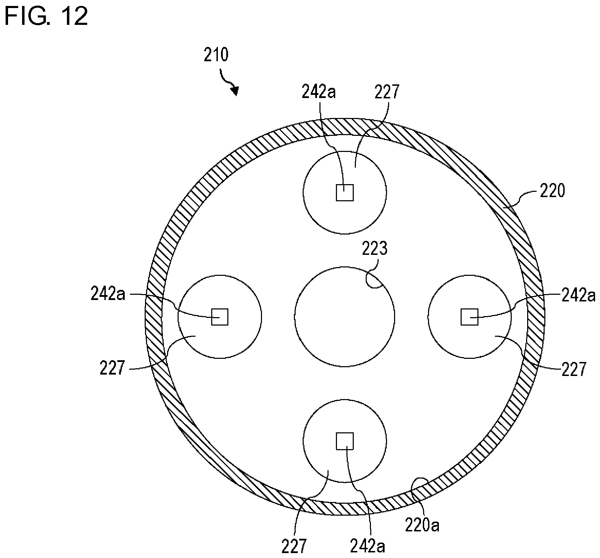

Figs. 11 and 12 are cross-sectional views schematically showing a configuration of a fluid sterilization apparatus 210 according to the second embodiment, and Fig. 12 shows an A-A cross section of Fig. 11. The fluid sterilization apparatus 210 includes a straight tube 220, an inflow pipe 231, an outflow pipe 232, a plurality of first light sources 240a, and a plurality of second light sources 240b. The fluid sterilization apparatus 210 differs from the first embodiment in that the inflow pipe 231 and the outflow pipe 232 are positioned on the central axis of the straight tube 220, thereby forming a straight flow passage 212 instead of an L-shaped flow passage. A description will be given of this embodiment, highlighting difference from the first embodiment.

The straight tube 220 extends from the first end 221 to the second end 222. The first end 221 is provided with a first end face 221a perpendicular to the longitudinal direction of the straight tube 220 and an inflow port 223 positioned near the center of the first end face 221a. The first end face 221a is provided with a plurality of first windows 227 for transmitting the ultraviolet light from the first light source 240a. The inflow pipe 231 extending in the longitudinal direction of the straight tube 220 is fitted to the inflow port 223. The inflow pipe 231 causes the fluid to flow in in the longitudinal direction of the straight tube 220 and inhibits turbulence from being produced in the flow in the flow passage 212.

The second end 222 is configured similarly to the first end 221. The second end 222 is provided with a second end face 222a perpendicular to the longitudinal direction of the straight tube 220 and an outflow port 224 positioned near the center of the second end face 222a. The second end face 222a is provided with a plurality of second windows 228 for transmitting the ultraviolet light from the second light source 240b. The outflow pipe 232 extending in the longitudinal direction of the straight tube 220 is fitted to the outflow port 224. The outflow pipe 232 causes the fluid to flow in in the longitudinal direction of the straight tube 220 and inhibits turbulence from being produced in the flow in the flow passage 212.

The first light sources 240a include a plurality of first light emitting devices 242a and a plurality of first substrates 244a. As shown in Fig. 12, the plurality of first light emitting devices 242a are positioned in four directions to surround the inflow port 223 and mounted to the first substrates 244a. Each of the plurality of first light emitting devices 242a emits ultraviolet light toward the interior of the straight tube 220 in the longitudinal direction of the straight tube 220 via the associated first window 227.

In the illustrated example, the first light emitting devices 242a are provided at four locations but the first light emitting devices 242a may be provided in three or fewer locations or in five or more locations. It is preferable that the plurality of first light emitting devices 242a be arranged at regular intervals so as to irradiate the entire fluid flowing in the flow passage 212. By positioning the first light emitting devices 242a at regular intervals so as to surround the inflow port 223, the first light sources 240a are capable of radiating ultraviolet light in a light intensity distribution in which the ultraviolet light intensity is higher near the center of the straight tube 220 and the ultraviolet light intensity is lower near the inner wall surface 220a of the straight tube 220.

The second light sources 240b include a plurality of second light emitting devices 242b and a plurality of second substrates 244ab and configured similarly to the first light sources 240a. The plurality of second light emitting devices 242b are positioned in four directions to surround the outflow port 224 and mounted to the second substrates 244b. Each of the plurality of second light emitting devices 242b emits ultraviolet light toward the interior of the straight tube 220 in the longitudinal direction of the straight tube 220 via the associated second window 228. Like the first light sources 240a, the second light sources 240b radiate ultraviolet light in a light intensity distribution in which the ultraviolet light intensity is higher near the center of the straight tube 220 and the ultraviolet light intensity is lower near the inner wall surface 220a of the straight tube 220.

The inner diameter of the straight tube 220 and the average flow rate of the fluid flowing in the flow passage 112 are adjusted so that the fluid flowing in the flow passage 212 is in a laminal flow state. This results in a flow rate distribution in which the flow rate of the fluid flowing near the central axis of the straight tube 220 is relatively high and the flow rate of the fluid flowing near the inner wall surface 220a of the straight tube 220 is relatively low. The fluid having such a flow rate distribution is irradiated with ultraviolet light from the first light sources 240a and the second light sources 240b of an intensity distribution in which the ultraviolet intensity near the center of the straight tube 220 is higher and the ultraviolet intensity near the inner wall surface 220a is lower. Thus, according to this embodiment, as in the case of the first embodiment, the sterilization efficiency for the fluid is improved by irradiating the fluid in a laminal flow state with ultraviolet light of an intensity distribution corresponding to the flow rate distribution of the laminal flow state.

In further accordance with this embodiment, the inflow port 223 and the outflow port 224 are positioned on the central axis of the straight tube 220. Therefore, turbulence and eddies are inhibited from being produced in the fluid flowing in the flow passage 212. Since the light sources 240a, 240b are positioned at both the inflow port 223 and the outflow port 224, the amount of ultraviolet energy affecting the fluid is increased as compared to the case of irradiating the fluid with ultraviolet light from only one port so that the sterilization efficiency for the fluid is improved.

In one variation, the light source may be positioned only at one of the inflow port 223 and the outflow port 224. The light sources 240a and 240b may be provided in the interior of the straight tube 220. Where the light sources 240a and 240b are provided in the interior of the straight tube 220, the light sources 240a and 240b are attached to the end faces 221a and 222b of the straight tube 220, and a cover member that transmit ultraviolet light is provided so as to prevent the light sources from being in direct contact with the fluid flowing in the flow passage 212.

Described above is an explanation based on an exemplary embodiment. The embodiment is intended to be illustrative only and it will be understood by those skilled in the art that various design changes are possible and various modifications are possible and that such modifications are also within the scope of the present invention.

The fluid sterilization apparatus 10 according to the embodiments is described as a apparatus for irradiating the fluid with ultraviolet light so as to sterilize the fluid. In one variation, the inventive fluid sterilization apparatus may be used for a purification process for decomposing organic substance included in a fluid by using ultraviolet irradiation.

In another variation, a straightener may not be provided in the middle of the above-described flow passage formed by the straight tube, and, more specifically, at the inflow port or at a position upstream of the inflow port. The straightener may have a function of straightening the flow of the fluid flowing in the flow passage and turning the fluid into a laminal flow. By providing the straightener, a laminal flow state characterized by less turbulence is formed to enhance the sterilization effect.

In one variation, the light source may be provided with an adjusting mechanism for adjusting the intensity distribution of ultraviolet light emitted by the light emitting device. The adjusting mechanism may include a transmissive optical element such as a lens or a reflective optical element such as a concave mirror. The adjusting mechanism may configure the intensity distribution of the ultraviolet light output from the light source to correspond to the flow rate distribution of the laminal state by adjusting the intensity distribution of the ultraviolet light from the light emitting device. By providing such an adjusting mechanism, the fluid is irradiated with ultraviolet light of an intensity distribution suitable for a mode of flow of the fluid and the sterilization efficiency is further enhanced.

The fluid sterilization apparatus 10 according to the embodiments can be used for sterilization of a variety of types of fluid other than the cutting fluid for machine tools, culture fluid for hydroponic culture, and Japanese sake. For example, proliferation of bacteria that affect the growth of fish adversely is prevented by sterilizing water in a water tank for keeping fish in an aquarium, farm, and home. Further, by cleaning sheets used in hospitals, accommodation facilities, homes, etc. such that the sheets are sterilized along with the cleaning liquid, pathogenic bacteria can be killed, and infection is prevented from spreading. Also, the detergent for dishwashers, skin lotion, and the like contains aromatic compounds in the form of effective ingredients, and bathwater in circulating hot water bath contains sebum components from human bodies in large amounts, but the fluid sterilization apparatus 10 according to the embodiment can sterilize these types of fluid efficiently.

### [DESCRIPTION OF THE REFERENCE NUMERALS]

10 ... fluid sterilization apparatus, 12 ... flow passage, 20 ... straight tube, 21 ... first end, 22 ... second end, 23 ... inflow port, 24 ... outflow port, 40 ... light source, 42 ... light emitting device, 140 ... light source, 210 ... fluid sterilization apparatus, 212 ... flow passage, 220 ... straight tube, 221 ... first end, 222 ... second end, 223 ... inflow port, 224 ... outflow port, 240a ... first light source, 240b ... second light source

### [INDUSTRIAL APPLICABILITY]

The present invention can be used in a fluid sterilization apparatus for sterilizing a fluid by irradiating the fluid with ultraviolet light.

## Claims

1. A fluid sterilization apparatus comprising:
a flow passage for causing a fluid to flow; and
a light source that irradiates the fluid flowing in the flow passage with ultraviolet light of a wavelength of 290∼310 nm.

2. The fluid sterilization apparatus according to claim 1, wherein
the fluid contains an aromatic compound.

3. The fluid sterilization apparatus according to claim 1 or 2, wherein
the flow passage includes a straight tube extending in a longitudinal direction,
the fluid flows in the flow passage in a laminal flow state, and
the light source includes a light emitting device that emits ultraviolet light of a wavelength of 290∼310 nm and radiates ultraviolet light in an intensity distribution in which an ultraviolet light intensity near a center in a cross section of the flow passage perpendicular to the longitudinal direction is higher than an ultraviolet light intensity around.

4. The fluid sterilization apparatus according to any one of claims 1 through 3, wherein
the fluid contains a cutting fluid for a cutting work.

5. The fluid sterilization apparatus according to any one of claims 1 through 3, wherein
the fluid contains a culture fluid for hydroponic culture.

6. The fluid sterilization apparatus according to any one of claims 1 through 3, wherein
the fluid contains Japanese sake in the process of manufacturing.

7. A fluid sterilization method comprising:
irradiating a fluid flowing in a flow passage with ultraviolet light of a wavelength of 290∼310 nm.
